# EUROPEAN PATENT SPECIFICATION

(11) **EP 0 505 316 B1**
(45) Date of publication and mention of the grant of the patent: **01.05.1996**
(21) Application number: 92670001.4
(22) Date of filing: 11.02.1992
(51) Int. Cl.: C07C 265/12, C07C 271/30, C07C 275/42

(54) **Diisocyanates, diurethanes and diureides from dehydroabietic acid**
Dehydroabietin-Säure, Diisocyanate, Diurethan und Diureide
Diisocyanates, diurethanes et diureides d'acide dehydroabietique

(30) Priority: 11.02.1991 PT 96748
(43) Date of publication of application: 23.09.1992
(73) Proprietor: INSTITUTO NACIONAL DE ENGENHARIA E TECNOLOGIA INDUSTRIAL (INETI), P-1699 Lisboa Codex (PT)
(72) Inventor: Carvalheiro, Bárbara Manuela Silva Gigante, P-1600 Lisboa (PT); Lobo, Ana Maria Felix Trindade, P-1900 Lisboa (PT); Prabhakar, Alberto Sundaresan, P-1900 Lisboa (PT); Curto, Maria Joao Marcelo, P-2790 Oeiras (PT)
(74) Representative: Arantes e Oliveira, Joao de

(56) References cited:
- CHEMICAL AND PHARMACEUTICAL BULLETIN. vol. 23, no. 12, 1975, TOKYO JP pages 3189-3202. AKIRA TAHARA 'Diterpenoids.XXXVIII.Conversion of 1-abietic acid into steroidal skeletons:formation of the D-ring'
- CHEMICAL ABSTRACIS, vol. 49, no. 4, 25 February 1955, Columbus Ohio, US; EIJI OCHIAI ET AL. 'Azaditerpenoids. i.Nitrogen derivatives of dehydroabietic acid' & J. PHARM.SOC.JAPAN vol. 74, 1954, pages 203-206

## Description

This invention relates to derivatives of dehydroabietic acid useful for polymers production and to processes for preparing them.

The invention provides dehydroabietic acid derivatives having the general formula (I) 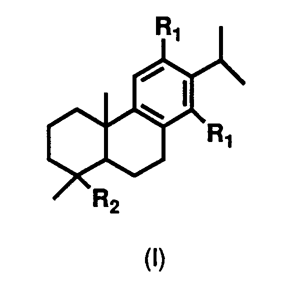
where R₁ represents an isocyanate, urethane or ureide group and R₂ represents a carboxylic acid, a salt or ester thereof, or amino group.

Dehydroabietic acid (II) is available in abundance at low cost and attempts have been made to prepare derivatives having useful properties. For example, Japanese patent application JP-90-104565 describes the preparation of amino derivatives of dehydroabietic acid for possible use in the production of nylon and polyurethanes. However, the process disclosed involves many steps, is costly and has therefore not been used commercially.

The compounds of the invention having the general formula (I) can be prepared from dehydroabietic acid, having the formula (II), obtained by dehydrogenation of rosin or abietic acid by known methods (see, for example, E. E. Fleck, US Patent 2,239,555, Apr. 22, 1941, and M. Ishigami and Y. Inoue, Yukagaku, 1974, **23**, 482) or by isolation from commercial disproportionated rosin (see, for example, N. J. Halbrook and R. Lawrence, US Patent 3,579,571, May 18, 1971), whose main component is dehydroabietic acid (II). 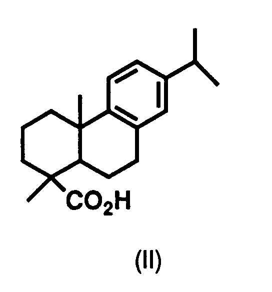
Nitration of dehydroabietic acid or one of its esters or salts produces the dinitro derivatives of general formula (III) (see, for example, L. F. Fieser and W. P. Campbell, J. American Chemical Society , 1938, **60**, 159). 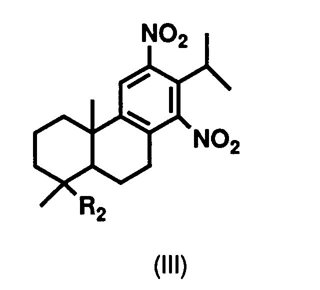
Catalytic hydrogenation of the dinitro derivatives (III) (see, for example, E. R. Littmann, J. American Chemical Society, 1938, **60**,1419) or its reduction (see, for example, A. Tahara , M. Shimagaki, M. Itoh, Y. Harigaya and M. Ohta, Chemical Pharmaceutical Bulletin, 1975, **23**, 3189) produces the diamino derivatives of general formula (IV). 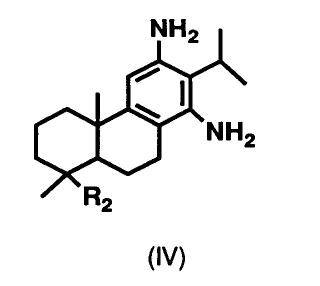
The new diisocyanate derivatives of general formula (V), where **R₂** represents a carboxylic acid, a salt or ester thereof, or amino group, are obtained by standard carbonylation procedures (see, for example, H. J. Twitchett, Chemical Society Reviews, 1974, **3**, 209) of the diamino derivatives. 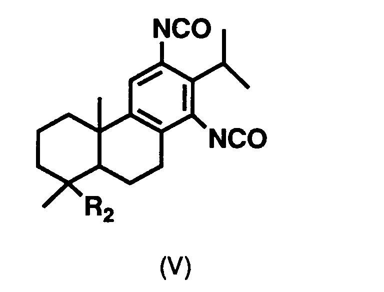
Reaction of the diisocyanate derivatives (V) with an alcohol or with an amine, using methods well established in the literature (see, for example, A. Farkas and G. A. Mills, Advanced Catalysis, 1962, **13**, 392) produce new urethane and diureide derivatives, respectively. Thus, new urethane derivatives of general formula (VI) where **R₂** represents a carboxylic acid, a salt or ester thereof, or amino group and **R₃** a chain with 1-27 carbon atoms, 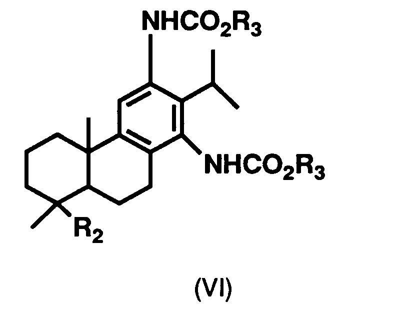
are obtained as a white or light pink powder, are produced from (V) by treatment with excess of an alcohol, where the alcohol is primary or secondary, aromatic or aliphatic, linear or non-linear, saturated or unsaturated, with up to 27 carbon atoms, in the presence or absence of an inert solvent, at room temperature or by heating at temperatures in the range 30-80°C, with a high degree of conversion.

New diureide derivatives of general formula (VII) where **R₂** represents a carboxylic acid, a salt or ester thereof, or amino group and **R₃** a chain with 1 - 27 carbon atoms, are produced from (V) by using excess of an amine, where the amine is primary or secondary, aromatic or aliphatic, linear or non-linear, saturated or unsaturated, with up to 27 carbon atoms, in the presence or absence of an inert solvent, at room temperature or by heating at temperatures in the range of 30-80°C. 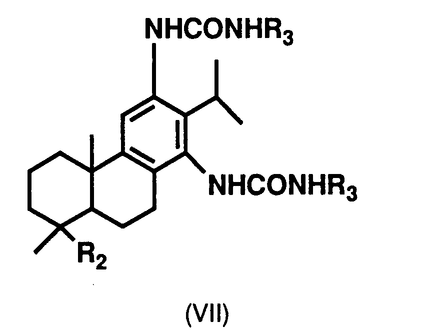
The invention compounds may be useful as industrial monomers for polymer production and the polymers and copolymers prepared from these resin acids derivatives are expected to show improved physical properties when compared with those of commercial polymers where such derivatives are not included (see, for example, John Lewis and Glen Hendrick, U.S. Pat. 3,702,338, Nov 7, 1972).

The following Examples illustrate the invention. The invention of one of the new diisocyanates is described in detail in Example 1. For convenience, the whole process is described starting from rosin, since it is an appropriate starting material for industrial application. Examples 2, 3 and 4 describe the production of new diurethane and diureide derivatives.

### EXAMPLE 1

This Example illustrates the preparation of methyl 12,14-diisocyano-dehydroabietate (I, R₁=NCO, R₂=CO₂Me).

Rosin (20 g) was methylated with excess diazomethane in diethyl ether:methanol (9:1) to afford a mixture of methylated resin acids (21 g) containing at least 60% methyl abietate. Upon purification to remove oxidised products, a clear gum (18,4 g) was obtained which was dehydrogenated with 5% palladium on charcoal (2% by weight) at 210-220°C for 2.5 hours, then dissolved in dichloromethane and filtered to remove the catalyst to afford a clear gum (17,5 g) containing at least 60% methyl dehydroabietate. Methyl dehydroabietate (III) (10 g) was nitrated with a mixture of concentrated sulphuric and nitric acids (6:4). The reaction mixture was washed with water and cold diethyl ether to produce methyl 12,14-dinitrodehydroabietate (10 g, 90%).

To methyl 12,14-dinitrodehydroabietate (10 g) dissolved in toluene:dichloromethane (3:1) were added a solution of hydrazine hydrate (40 ml) and a freshly prepared suspension of Raney nickel (70 ml). After 24 hours at 30°C, the catalyst was removed by filtration and the filtrate concentrated at reduced pressure to give, upon purification, methyl 12,14-diaminodehydroabietate (8 g, 94%).

To methyl 12,14-diaminodehydroabietate (8 g) dissolved in toluene (120 ml) and cooled in an ice bath was slowly added a 20% solution of phosgene in toluene (400 ml). After 0.5 hours at room temperature, the solvent was removed at reduced pressure to afford methyl 12,14-diisocyano-dehydroabietate (9,2 g, 100%). The structure of this new compound was established by IR, ¹³C NMR and MS: νₘₐₓ (CCl₄) 2275 (N=C=O), 1730 (C=O, ester) cm⁻¹; [¹³C] δ (CDCl₃) C-22 or C-23 (125.28 or 125.27) ppm; m/z 396.2042 [M⁺] (C₂₃H₂₈N₂O₄ requires 396.2049).

### EXAMPLE 2

This Example illustrates the preparation of methyl 12,14-dimethylurethane-dehydroabietate (I, R₁ =NHCOCH₃, R₂=CO₂Me).

An excess dry methanol (3 ml) was added to methyl 12,14-diisocyano-dehydroabietate (3 g) at room temperature with stirring for ca. 1 hour. Upon distillation of methanol, a light pink powder (3,5 g) was obtained which was purified by chromatography to afford methyl 12,14-dimethylurethane-dehadroabietate (2,2 g ; 64%). The structure of the new compound was established by IR, ¹H NMR and MS: νₘₐₓ (CHCl₃) 3412 (N-H), 1720 cm⁻¹ (strong broad band, C=O ester and urethane); [¹H] δ (CDCl₃) 3.655 (3H, *s*, 12- or 14-NCOOMe), 3.760 (6H, *s*, 12-or 14-NCOOMe and COOMe), 5.868 e 6.423 (2H, 2*sl*, 12- and/or 14-NH) (exchange with NaOD/D₂O), 7.527 (1H, *s*, 11-H) ppm; m/z 460.2560 (C₂₅H₃₆N₂O₆ requires 460,2564).

### EXAMPLE 3

This Example illustrates the preparation of methyl 12,14-dibutylurethane-dehydroabietate (I, R₁=NHCO(CH₂)₃CH₃), R₂=CO₂Me).

An excess dry *n*-butanol (4 ml) was added to methyl 12,14-diisocyano-dehydroabietate (2.5 g) at room temperature with stirring for ca. 1 hour. Upon distillation of the excess of alcohol, a reddish powder was obtained which after purification afforded methyl 12,14-dibutylurethane-dehydroabietate (1,3 g; 50%). The structure of the new compound was established by IR, ¹H NMR and MS: νₘₐₓ (KBr) 3420 (N-H), 1725-1705 cm⁻¹ (C=O, ester and urethanes); [¹H] δ (CDCl₃) 1.181 (3H, *sl* , 10-Me), 1.198 and 1.273 (6H, 2 *sl*, 26-H and/or 26′-H), 1.248 (3H, *s* , 4-Me), 1.423 (6H, 2*d* , *J* 7, 15-Me₂), 3.655 (3H, *s* , COOMe), 4.077 (6H, *sl* and *t* , 12-NH, 14-NH and 2xOCH₂), 7.234 (1H, *s* ,11-H ) ppm; m/e 544.3551 (C₃₁H₄₈N₂O₆ requires 544.3512).

### EXAMPLE 4

This Example illustrates the preparation of methyl 12,14-diisopropylureide-dehydroabietate (I, R₁= NHCONHCH(CH₃)₂, R₂=CO₂Me).

Isopropylamine (3 ml) was added to methyl 12,14-diisocyano-dehydroabietate (2 g) with stirring for 1 hour. Distillation of the excess amine produced a gum (3,2 g) containing methyl 12,14-diisopropylureide-dehydroabietate (1,5 g, 47%), whose structure was established by IR, ¹H NMR and MS: νₘₐₓ (CHCl₃) 3410 (N-H), 1720 (C=O, ester), 1655 (C=O, urea) cm⁻¹; [¹H] δ (CDCl₃) 1.080 (12H, *m* , 23-Me₂ and 23′-Me₂), 1.199 (3H,*s* , 10-Me), 1.266 (3H, *s* , 4-Me), 1.114 (6H, 2*d* , *J* 7, 15-Me₂), 3.661 (3H, *s* , COOMe), 3.990 (2H, *m* , 23-H), 4.365 (2H, *sl* , 2xCO-NH-*i*Pr) (exchange with D₂O), 5.884 and 6.130 (2H, 2 *sl* ,12-NH and/or 14-NH) (exchange with D₂O), 7.226 (1H, *s* ,11-H) ppm; m/e 514.3536 (C₂₉H₄₆N₄O₄ requires 514.3519).

## Claims

1. Compounds of general formula (I) 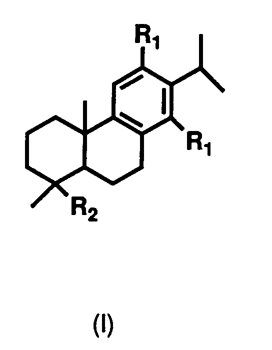 where **R₁** represents an isocyanate, urethane or ureide group and **R₂** represents a carboxylic acid, a salt or ester thereof, or amino group;

2. A process for preparing compounds according to claim 1 wherein **R₁** represents an isocyanate group, which comprises nitration of dehydroabietic acid or a salt, ester or amine thereof, followed by catalytic hydrogenation or reduction, and carbonylation of the resulting diamine.

3. A process for preparing compounds according to claim 1 wherein **R₁** represents a urethane group, which comprises reaction of a diisocyanate obtainable by the process of claim 2 with an alcohol.

4. A process according to claim 3, where the alcohol is primary or secondary, aromatic or aliphatic, linear or non-linear, saturated or unsaturated, with up to 27 carbon atoms.

5. A process for preparing compounds according to claim 1 wherein **R₁** represents a ureide group, which comprises reaction of a diisocyanate obtainable by the process of claim 2 with an amine.

6. A process according to claim 5, where the amine is primary or secondary, aromatic or aliphatic, linear or non-linear, saturated or unsaturated, with up to 27 carbon atoms.

## Patentansprüche

1. Verbindungen der allgemeine Formel(I) 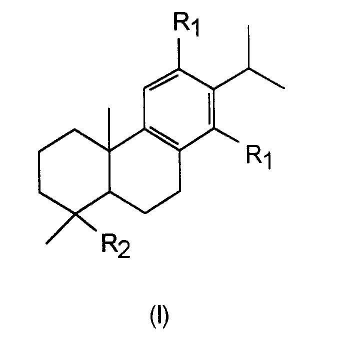 wobei R₁ eine Isocyanat-, Urethane- oder Ureidegruppe, und R₂ eine Carbonsäure, ein deren Salz oder Ester, oder eine Aminogruppe darstellt.

2. Verfahren für Herstellung von Verbindungen gemäß Anspruch 1, wobei R₁ eine Isocyanatgruppe darstellt, dadurch gekennzeichnet, daß die Nitrierung von Dehydroabietinsäure oder einen deren Salz, Ester oder Amin, dann die katalytisch Hydrierung oder Reduktion und Carbonylierung des entstandenes Diamin umfaßt.

3. Verfahren für Herstellung von Verbindungen gemäß Anspruch 1, wobei R₁ eine Urethanegruppe darstellt, dadurch gekennzeichnet, daß die Umsetzung von einem durch das Verfahren der Anspruch 2 herstellbar Diisocyanat mit einem Alkohol umfaßt.

4. Verfahren nach Anspruch 3, dadurch gekennzeichnet, daß der Alkohol primär oder sekundär, aromatisch oder aliphatisch, gerade oder verzweigt, gesättigt oder ungesättigt ist, und bis zu 27 Kohlenstoffatomen enthält.

5. Verfahren für Herstellung von Verbindungen gemäß Anspruch 1, wobei R₁ eine Ureidegruppe darstellt, dadurch gekennzeichnet, daß die Umsetzung von einem durch das Verfahren der Anspruch 2 herstellbar Diisocyanat mit einem Amin umfaßt.

6. Verfahren nach Anspruch 5, wobei das Amin primär oder sekundär, aromatisch oder aliphatisch, gerade oder verzweigt, gesättigt oder ungesättigt ist, und bis zu 27 Kohlenstoffatomen enthält.

## Revendications

1. Composés avec la formule générale (I) 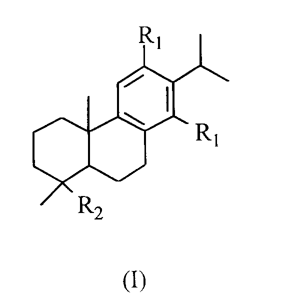 où R₁ représente un groupe isocyanate, uréthane ou ureide et R₂ représente un acide carboxylique, un sel, un ester ou un groupe amine;

2. Un procédé pour la préparation de composés selon la revendication 1 où R₁ représente un isocyanate, comprenant la nitration de l'ácide desidroabiétique, un sel, un ester ou une amine suivi de hydrogénation catalytique ou de réduction, et carbonilation de la diamine obtenue.

3. Un procédé pour la préparation de composés selon la revendication 1 où R₁ représente un un groupe uréthane, comprenant la reaction d'un isocyanate, obtenu selon le procédé de la revendication 1, avec un alcohol.

4. Un procédé selon la revendication 3 où l'alcool est primaire ou sécondaire, aromatique ou aliphatique, linéaire ou non-linéaire, saturé ou insaturé, avec jusqu'à 27 atomes de carbone.

5. Un procédé pour la préparation de composés selon la revendication 1 où R₁ représente un un groupe ureide, comprenant la reaction d'un isocyanate, obtenu selon le procédé de la revendication 1, avec une amine.

6. Un procédé selon la revendication 5 où l'amine est primaire ou sécondaire, aromatique ou aliphatique, linéaire ou non-linéaire, saturé ou insaturé, avec jusqu'à 27 atomes de carbone.
